# DEMANDE DE BREVET EUROPEEN

(11) **EP 0 566 484 A1**
(43) Date de publication de la demande: **20.10.1993**
(21) Numéro de dépôt: 93400968.9
(22) Date de dépôt: 14.04.1993
(51) Int. Cl.: C07D 211/18, C07D 211/70, A61K 31/445, C08L 55/02

(54) **(N-(2-(naphthyl-2)-éthyl)-4-(3-trifluorométhylphényl) pipéridine, procédé pour sa préparation et compositions pharmaceutiques la contenant**

(30) Priorité: 17.04.1992 FR 9204821
(71) Demandeur: SANOFI, F-75008 Paris (FR)
(72) Inventeur: Baroni, Marco, I-20010 Vanzago (Milano) (IT); Guzzi, Umberto, I-20100 Milano (IT); Arnone, Michèle, F-31520 Ramonville Saint Agne (FR); Soubrie, Philippe, F-34270 Saint Mathieu de Treviers (FR)
(74) Mandataire: Gillard, Marie-Louise

(57) **Abrégé**

L'invention a pour objet la 1-[2-(2-naphtyl)éthyl]-4-(3-trifluorométhylphényl)pipéridine de formule 1 et ses sels d'addition d'acides, utiles dans le traitement du dysfonctionnement sexuel chez les mammifères. Le nouveau composé peut être préparé par exemple par hydrogénation catalytique du dérivé 1,2,3,6-tétrahydropyridinique correspondant.

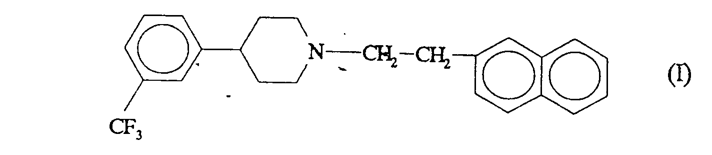

## Description

La présente invention concerne un nouveau dérivé de la pipéridine ainsi que le sels d'addition d'acides de ce dérivé, le procédé pour sa préparation et les compositions pharmaceutiques le contenant.

Plusieurs dérivés de la pipéridine sont connus dans l'art antérieur. En particulier, la demande de brevet anglais GB-A-2 083 476 revendique des 4-aryl-pipéridines ou -tétrahydropyridines douées d'activité psychotrope et agoniste de la dopamine de formule (A) 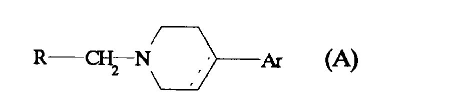
dans laquelle la ligne pointillée représente rien ou une liaison carbone-carbone, R représente un groupe indol-3-yle, napht-1-yle, napht-2-yle, ou benzot hien-3-yle ; etAr représente un groupe phényle éventuellement substitué.

La demande de brevet internationale WO-A-89/11476 revendique des 2-aminothiazoles à activité antipsychotique qui peuvent être substitués par un groupe arylpipéridinoéthyle.

La demande européenne EP-A-0 372 776 décrit des N-alkyl arylpipéridines, ayant une activité antipsychotique, dans lesquelles le groupe alkyle lié à l'atome d'azote porte un substituant phényle, phényle substitué ou hétéroaryle.

La demande internationale WO-A-91/09594 revendique l'utilisation d'une large classe de composés y compris les N-arylalkyl- ou N-hétéroaryl-alkyl 4-arylpipéridines pour le traitement de la schizophrénie et d'autres psychoses.

Enfin, le brevet européen EP-B-101 381 décrit et revendique des dérivés 4-(3-trifluorométhylphényl)-1,2,3,6-tétrahydropyridines à activité anorexigène y compris le composé de formule (B) suivante 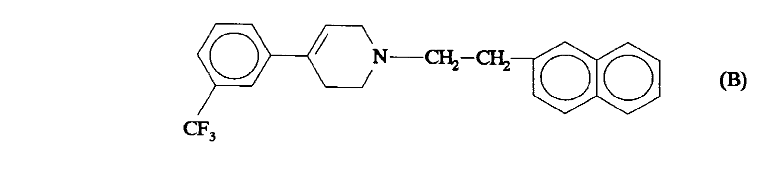
pour lequel différentes autres utilisations thérapeutiques ont été décrites dans les demandes de brevet européen EP-A-0 369 887 (utilisation dans les troubles anxio-dépressifs), EP-A-0 412 901 (utilisation dans les troubles de la motricité intestinale) et EP-A-0 458 696 (utilisation dans les troubles cérébraux et neuronaux).

On a maintenant trouvé que le composé de formule (I) suivante 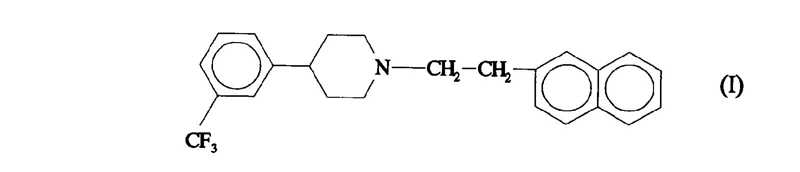
qui diffère du composé (B) par l'absence de la double liaison dans le noyau pipéridinique, présente un bon profil d'activité dans les tests de comportement sexuel chez les animaux à des doses beaucoup plus faibles par rapport au composé (B) et que, contrairement au composé (B) il est complètement inactif ou très peu actif comme antidépressif/anxiolytique et il n'a aucune activité neurotrophe/ neuroprotectrice.

Un premier objet de la présente invention est donc le composé de formule (I) ci-dessus ainsi que ses sels d'addition d'acides.

Le composé de formule (I) est en effet un compose basique qui peut former des sels d'addition avec les divers acides organiques ou inorganiques.

Bien que pour l'administration aux animaux ou pour la préparation des compositions pharmaceutiques, ces sels doivent être pharmaceutiquement acceptables, il peut être convenable d'isoler du mélange réactionnel le composé de formule (I) sous forme d'un sel d'addition avec un acide pharmaceutiquement non acceptable, le convertir en une base libre par traitement avec un réactif alcalin et éventuellement convertir la base libre ainsi obtenue en un sel d'addition pharmaceutiquement acceptable.

Tant les sels d'addition du composé (I) pharmaceutiquement acceptables que ceux qui ne le sont pas font donc partie de la présente invention. Ils sont facilement préparés par traitement du composé (I) sous forme de base libre avec une quantité au moins équimolaire de l'acide organique ou inorganique convenablement choisi, dans un solvant aqueux ou organique, tel que l'éthanol ou l'isopropanol, suivi par l'évaporation à sec du solvant.

Les acides qui sont utilisés pour la préparation des sels d'addition pharmaceutiquement acceptables du composé (I) sont ceux qui forment des sels d'addition non toxiques, à savoir des sels qui contiennent des anions acceptables du point de vue pharmacologique, tels que le chlorhydrate, le bromhydrate, l'iodhydrate, le nitrate, le sulfate, l'hydrogénosulfate, le phosphate, l'acétate, le lactate, le succinate, le tartrate, le fumarate, le maléate, le gluconate, le D-glucarate, l'iséthionate, le méthanesulfonate, le p-toluènesulfonate, le pamoate, l'angélate, etc.

Le composé de formule (I) peut être préparé selon l'une quelconque des méthodes suivantes :
(a) par réaction de la 4-(3-trifluorométhylphényl)pipéridine de formule (II) 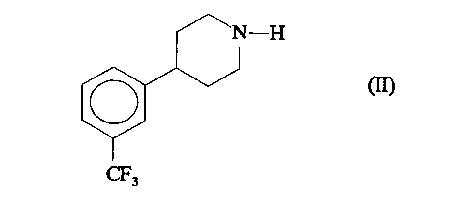
   avec un composé de formule (III) 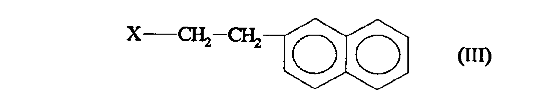
   où X représente un atome de chlore, brome, iode ou un groupe partant tel qu'un groupe méthanesulfony- loxy ou p-toluènesulfonyloxy,
   éventuellement et avantageusement en présence d'une base conventionnelle pour neutraliser l'acide HX qui se forme au cours de la réaction ;
(b) par réaction de la 4-(3-trifluorométhylphényl)pipéridine de formule (II) avec un dérivé fonctionnel de l'acide (IV) 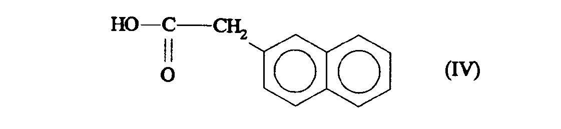
   suivie par la réduction du produit intermédiaire ainsi obtenu ; ou
(c) par réduction de la double liaison dans le dérivé 1,2,3,6-tétrahydropyridinique correspondant de formule
(B) ci-dessus.

Dans la méthode de préparation (a), on utilise de préférence un solvant organique polaire tel qu'un alcanol contenant de 1 à 5 atomes de carbone, par exemple méthanol, éthanol, butanol ou pentanol ; un éther cyclique, par exemple le dioxanne ou le tétrahydrofuranne ; une cétone aliphatique, par exemple l'acétone, la méthylé- thylcétone, la méthylisobutylcétone, etc. ; les sulfoxydes alkyliques, par exemple le diméthylsulfoxyde et le diéthylsulfoxyde ou les dialkylamides, par exemple le diméthylacétamide ou le diméthylformamide, etc.

De préférence la réaction est conduite en présence d'une base conventionnelle en quantité au moins équimolaire par rapport à l'acide qui se forme au cours de la réaction. Cette base peut être un carbonate ou un bicarbonate alcalin ou une base organique telle qu'une amine aliphatique tertiaire, en particulier la triéthyla- mine.

La température de réaction peut varier entre la température ambiante et 200°C et les temps de réaction varient en conséquence. En général, après 4 à 5 h à 100-150°C, la réaction est terminée et le composé (I) ainsi obtenu peut être isolé selon les techniques conventionnelles et éventuellement transformé en l'un de ses sels par simple salification.

Dans la première étape de la méthode de préparation (b), on fait réagir le composé de formule (II) avec un dérivé fonctionnel de l'acide (IV) en utilisant, comme dérivé fonctionnel approprié, l'acide libre activé, l'anhydride, un anhydride mixte, un ester actif ou un halogénure d'acide, de préférence le chlorure. Parmi les esters actifs, l'ester de p-nitrophényle est particulièrement préféré, mais les esters de méthoxyphényle, de trityle, de benzhydryle et similaires sont également convenables.

La température de réaction peut varier entre -10°C et la température d'ébullition du solvant employé, mais en général on opère à une température comprise entre la température ambiante et 50°C. Il peut être préférable de conduire la réaction à basse température lorsqu'elle est exothermique, comme dans le cas où on utilise le chlorure en tant que dérivé fonctionnel de l'acide de formule (IV).

Comme solvant de réaction, on utilise de préférence un alcool, tel que le méthanol ou l'éthanol, ou un solvant halogéné tel que le chlorure de méthylène, le dichloroéthane, le chloroforme et similaires, mais d'autres solvants organiques compatibles avec les réactifs employés, par exemple le dioxane, le tétrahydrofuranne ou un hydrocarbure tel que l'hexane, peuvent être également employés.

La réaction peut être convenablement conduite en présence d'un accepteur de protons, par exemple d'un carbonate alcalin ou d'une amine tertiaire, dans le cas où un acide se libérerait pendant la réaction, mais cet accepteur de protons n'est pas indispensable pour l'obtention de l'amide intermédiaire.

Ce produit intermédiaire peut être isolé et caractérisé selon les techniques conventionnelles, mais il peut aussi être utilisé à l'état brut pour l'étape suivante de réduction.

Cette deuxième étape est conduite selon les modes opératoires connus en soi en utilisant comme agent de réduction l'hydrure d'aluminium ou un hydrure complexe de lithium et d'aluminium, tel que LiAIH₄, LiAIH(OCH₃)₃ et similaires. On opère en général dans un solvant inerte comme un éther, tel que l'éther diéthylique, le tétrahydrofuranne, le dioxane ou le 1,2-diméthoxyethane.

Selon un mode opératoire préféré, on opère avec une quantité équimolaire d'hydrure de lithium et d'aluminium, LiAIH₄, par rapport au composé intermédiaire obtenu dans l'étape précédente, à une température de 20-30°C dans de l'éther diéthylique et sous atmosphère inerte. Après environ 1 h, la réduction est complète et le composé de formule (I) est isole selon les techniques conventionnelles sous forme de base libre ou d'un de ses sels.

Selon la méthode de préparation (c), on obtient le composé (I) simplement par hydrogénation catalytique, de préférence en utilisant Pd/C comme catalyseur d'hydrogénation et de l'éthanol comme solvant réactionnel. La réaction peut être conduite à une température comprise entre la température ambiante et la température de reflux du solvant réactionnel. Lorsque la réaction d'hydrogénation est terminée, le catalyseur est éliminé par filtration et le produit (I) est obtenu par évaporation du solvant et cristallisation à l'aide d'un solvant convenable.

Le composé de départ de formule (II) est un produit connu dont la préparation est décrite par exemple dans la demande européenne EP-A-0 372 776, Préparation L.

L'acide de formule (IV) et ses dérivés fonctionnels sont également connus en littérature libre et le composé (B), comme il a été précisé ci-dessus, est décrit dans le brevet européen EP-B-101 381.

Dans ce qui suit, on décrit à titre d'exemple non limitatif, la préparation du composé (I) selon la méthode (c):

Un mélange de 5 g (0,012 mol) du chlorhydrate de 1-[2-(2-naphtyl)éthyl]-4-(3-trifluorométhylphényl)-1,2,3,6-tétrahydropyridine, 0,6 g de Pd/C 10 % et 130 ml d'éthanol à 95 % est chargé dans un appareil d'hydrogénation et hydrogéné, sous agitation, à 50°C pendant 3 h. Le catalyseur est alors éliminé par filtration et le filtrat est concentré à sec. Le résidu est trituré dans une petite quantité d'éther éthylique et le produit solide blanc qu'on obtient par filtration est cristallisé dans 120 ml d'éthanol absolu et séché en donnant 3,5 g (0,0083 mol) de chlorhydrate de 1-[2-(2-naphtyl)éthyl]-4-(3-trifluorométhylphényl)pipéridine. P.f. 296-298°C.

A partir du composé ainsi obtenu, par conversion en la base libre correspondante et salification avec l'acide approprié, on a préparé le fumarate, le p-toluènesulfonate, le succinate, le L(+)-tartrate et l'iséthionate correspondants.

Le composé de formule (I) de la présente invention ainsi que ses sels d'addition d'acides pharmaceutiquement acceptables, testé chez les rats selon la méthodologie décrite par A. Âgmo et Z. Picker dans Phar- macol. Biochem. Behav., 1990, 35 (2), 327-334, modifie de façon très favorable le comportement sexuel des animaux.

Plus particulièrement, on a trouvé que le composé de formule (I), chez le rat, augmente de façon très significative la motivation sexuelle du mâle (évaluée comme nombre de montes pendant un temps fixé) ainsi que la performance du rat inexpérimenté en activité copulatoire (en donnant tant une augmentation remarquable du pourcentage d'animaux copulants qu'une dimunition aussi remarquable de la latence d'éjaculation) à des doses qui ne donnent aucune activité anxiolytique/antidépressive ni neurotrophe/neuroprotectrice.

Le composé de formule (I) est aussi peu toxique et peut donc être utile comme médicament.

On envisage donc l'utilisation du composé de formule (I) et de ses sels d'addition d'acides pharmaceutiquement acceptables dans le traitement du dysfonctionnement sexuel chez les mammifères qui présentent ce dysfonctionnement et nécessitent un traitement, qui comprend l'administration d'une quantité efficace pour ce but d'au moins un composé de formule (I) ou d'un de ses sels pharmaceutiquement acceptables.

Le composé de formule (I) et ses sels d'addition d'acides pharmaceutiquement acceptables peuvent être administrés tant par voie orale que parentérale. En général, le principe actif selon la présente invention est plus préférablement administré à des doses comprises entre environ 1,0 mg et environ 200 mg par jour, même si des variations peuvent être nécessaires selon le poids et la condition du patient, sa réponse individuelle au médicament, la voie d'administration, le type particulier de formulation pharmaceutique choisie et le schéma de traitement.

Le composé de formule (I) et ses sels peuvent être administrés seuls ou en mélange avec des véhicules et/ou des additifs pharmaceutiquement acceptables, par voie orale ou parentérale, selon des schémas de traitement qui prévoient une administration quotidienne unique ou plusieurs administrations par jour.

Plus particulièrement, le nouveau composé de formule (I) ainsi que ses sels pharmaceutiquement acceptables peuvent être administrés dans une grande variété de formes de dosage : comprimés, gélules, poudres, suppositoires, suspensions ou solutions injectables, élixirs, sirops, etc.

La voie d'administration préférée pour le traitement du dysfonctionnement sexuel est la voie orale.

Pour cette administration on utilise, de préférence, un sel d'addition du composé de formule (I), on le mélange avec les additifs et les véhicules conventionnellement utilisés dans l'industrie pharmaceutique et on le formule sous forme de comprimés, comprimés sucrés, gélules, solutions ou suspensions.

Pour l'administration orale, on peut préparer des composés en utilisant comme véhicule pharmaceutiquement acceptable de la cellulose microcristalline, du lactose, du phosphate dicalcique, de la glycine, etc., en combinaison éventuellement avec des agents de désagrégation, tels que l'amidon (de maïs, de pomme de terre, de tapioca, etc.), l'acide alginique, etc., des agents de dispersion ou de mise en suspension, tels que la polyvinylpyrrolidone, le saccharose, la gélatine, etc. En outre, des agents lubrifiants, tels que le stéarate de magnésium, le talc ou le laurylsulfate de sodium, sont souvent très utiles dans la préparation des comprimés. Les comprimés peuvent aussi être traités de telle sorte qu'ils aient une activité prolongée ou retardée et qu'ils libèrent d'une façon continue une quantité prédéterminée de principe actif.

Les compositions solides du type ci-dessus peuvent également être utilisées pour la préparation de gélules et des poudres ou granules dispersibles dans l'eau.

Une préparation liquide sous forme de sirop ou d'élixir ou pour l'administration en gouttes peut contenir l'ingrédient actif conjointement avec un édulcorant, de préférence acalorique, du méthylparaben et du propyl- paraben comme antiseptiques, ainsi qu'un agent donnant du goût et un colorant approprié, dans un véhicule liquide.

Le principe actif peut être formulé également sous forme de micro-capsule ou de microémulsions, éventuellement avec un ou plusieurs supports ou additifs.

Le principe actif peut aussi être encapsulé dans une cyclodextrine (par exemple, a ou ¡3-cyclodextrine) pour augmenter sa solubilité.

Pour l'administration parentérale, les compositions pharmaceutiques selon l'invention contiennent, outre le principe actif, un véhicule ou un mélange de véhicules, aqueux ou non aqueux, stériles, acceptables du point de vue pharmaceutique.

Ces compositions pharmaceutiques peuvent aussi contenir des adjuvants tels que des agents stabilisants, mouillants, émulsionnants ou dispersants convenables.

Les compositions pharmaceutiques contenant comme principe actif, le composé de formule (I) ou un de ses sels d'addition pharmaceutiquement acceptables, représentent un autre objet spécifique de la présente invention.

Particulièrement préférées sont les compositions pharmaceutiques contenant d'environ 0,5 à environ 100 mg de principe actif par unité de dosage.

## Revendications

1. Composé de formule (I) 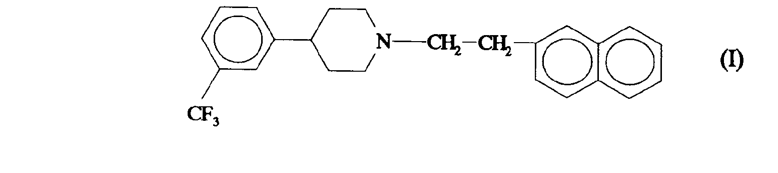
et ses sels d'addition d'acides.

2. Composé selon la revendication 1, où les sels d'addition d'acides sont pharmaceutiquement acceptables.

3. Procédé de préparation d'un composé de la revendication 1, caractérisé en ce qu'il comprend:
(a) la réaction de la 4-(3-trifluorométhylphényl)pipéridine de formule (II) 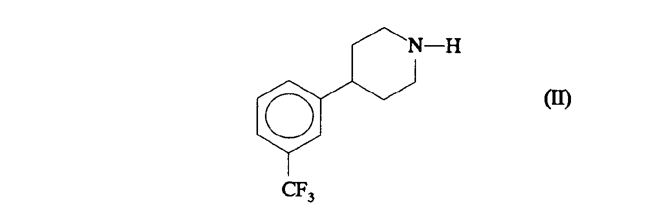
avec un composé de formule (III) 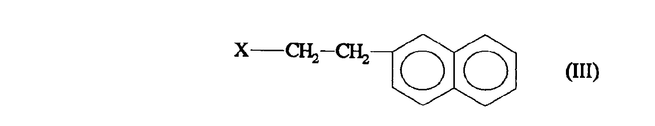
où X représente un atome de chlore, brome, iode ou un groupe partant tel qu'un groupe méthanesul- fonyloxy ou p-toluènesulfonyloxy, éventuellement en présence d'une base ;
ou
(b) la réaction de la 4-(3-trifluorométhylphényl)pipéridine de formule (II) avec un dérivé fonctionnel de l'acide (IV) 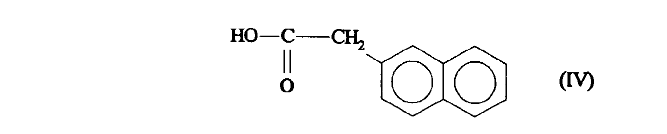
suivie par la réduction du produit intermédiaire ainsi obtenu ;
ou
(c) la réduction de la double liaison dans le dérivé 1,2,3,6-tétrahydropyridinique correspondant de formule (B) 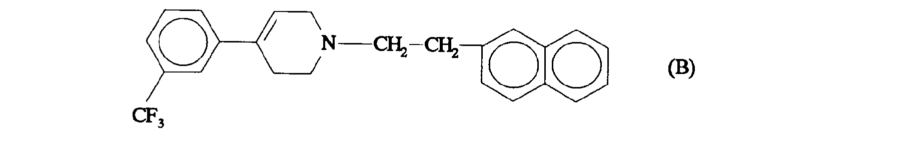
éventuellement suivies par la conversion dans le sel d'addition voulu.

4. Composition pharmaceutique contenant en tant que principe actif un composé selon la revendication 2.

5. Composition selon la revendication 4, caractérisée en ce qu'elle contient environ 0,5 à environ 100 mg de principe actif par unité de dosage.
